# EUROPEAN PATENT APPLICATION

(11) **EP 0 694 297 A1**
(43) Date of publication of application: **31.01.1996**
(21) Application number: 95304730.5
(22) Date of filing: 06.07.1995
(51) Int. Cl.: A61F 13/15

(54) **Training pants**

(30) Priority: 09.07.1994 GB 9413871; 13.08.1994 GB 9416384
(71) Applicant: SKIPPINGDALE PAPER PRODUCTS LIMITED, Scunthorpe, South Humberside DN15 8NN (GB)
(72) Inventor: Akesson, Mats Yngve, Grasby, Barnetby, DN38 6AU (GB)
(74) Representative: Walker, Antony James Alexander

(57) **Abstract**

Disposable pants comprise a front section (3), a rear section (4) and a crotch section (5) which connects the front and rear sections (3 and 4) together. The sides (6 and 7) of the crotch section (5) are cut away to define leg openings, and elastication (10, 11, 12 and 13) extends along the sides of each cut away so as to elasticate the leg openings and ensure a secure and fluid tight fit on a wearer. The elastication comprises four groups of elastic members (10, 11, 12 and 13); each of which extends diagonally across the crotch section and along a respective half of one of the two cut aways which define the leg openings.
another of the four groups of elastic members extends from a point on the said one side of the front section to a point on the said diametrically opposite side of the rear section along the rear half of the other of the two cut aways which define the leg openings;
yet another of the four groups of elastic members extends from a point on the side of the rear section directly opposite the said one side of the front section to a point on the diametrically opposite side of the rear section along the rear half of the said one of the two cut aways which define the leg openings; and
the last of the four groups of elastic members extends from a point on the said side of the rear section directly opposite the said one side of the front section to a point on the diametrically opposite side of the rear section along the front half of the said other of the two cut aways which define the leg openings.

## Description

The present invention relates to disposable underpants such as diapers, infant training pants and incontinence pants. More specifically the present invention relates to disposable underpants which are usually manufactured from continuous webs and continuous elastic threads in a continuous production process.

US-A-5055103 discloses disposable underpants comprising a water permeable topsheet, a water permeable backsheet and a water absorbent core sandwiched between the top and backsheets. In order to prevent excretions from passing through the backsheet a water permeable plastic film is bonded to the inner side of the backsheet. Elastication is provided around each of the leg openings and around the waist opening to retain the disposable underpants in place on a wearer and also to minimise undesirable leakage of excretions, particularly through the leg openings on each side of the crotch area.

The elastication which surrounds each of the leg openings is comprised of first and second groups of elastic threads which are bonded onto the backsheet as part of the manufacturing process. The first group of elastic threads extend down along the front edge of one of the leg openings to the bottom thereof, transversely across the crotch area and then back along the front edge of the opposite leg opening to the top thereof. The second group of elastic threads follows a similar path along the rear edge of each leg opening. Where the first and second groups of elastic threads meet at the bottom of the front and rear edges of each leg opening they intersect before crossing the crotch section to the opposite leg opening.

In any diaper, incontinence pants or infant training pants it is inevitable that if there is any leakage of fluids this will generally occur at the sides of the crotch section owing to the location and orientation of the urinary organs. For this reason it is generally considered desirable for the elastication in this region to be greater than elsewhere around the leg opening. The disposable underpants of US-A-5055103 achieve this objective by laying down the first and second groups of elastic threads onto the backsheet in such a way that their tensile stress gradually increases along each of the front and rear edges towards the bottom thereof.

However, the increased transile stress must not be transferred into the portions of the first and second elastic members running transversely across the crotch section as this may cause bunching of the absorbent pad. Bunching is considered undesirable as it affects the absorption proportions of the pad and may lead to leakage. It can also affect the fit and comfort of the disposable underpants on the wearer. In US-A-5055103 this problem is solved by not bonding these portions to the backsheet. Moreover, each portion is overrun between the points of intersection of the first and second elastic members so as to reduce the tension therein. From a production point of view though this does cause difficulties as the adhesive must be switched off each time the first and second elastic members are transversed across the crotch section.

It is an object of the present invention to provide disposable underpants which differ from that of US-A-5055103 and yet which provide a snug, secure and fluid tight fit around the wearers legs.

It is another object of the present invention to provide disposable underpants in which the elastic members which provide elastication around the leg openings also provide elastication around the hip area to further improve fit.

It is a further object of the present invention to provide disposable underpants which are not prove to bunching across the crotch section.

According to the present invention there is provided disposable underpants comprising a front section, a rear section, a crotch section which connects the front and rear sections together and the sides of which are cut away to define leg openings, and elastication which extends along the sides of each cut away so as to elastic the leg openings and ensure a secure and fluid tight fit on a wearer, characterised in that the elastication comprises four groups of elastic members each of which extends from a point on one side of the front section to a point diametrically opposite on one side of the rear section and each of which extends along a respective half of a respective one of the two cut aways which define the leg openings.

This arrangement of elastic members ensures that there is no change over in direction of the paths of the elastic members through the crotch section, as is the case in the known disposable underpants of US-A-5055103. In other words after passing around the edge of the leg opening each elastic member continues on its way at a relatively shallow angle thereto. As a consequence the tension of the elastication provided around each leg opening can be kept even and constant about its circumference whilst still ensuring a snug, secure and fluid tight fit around the wearers leg. It also ensures that there is no bunching of the absorbent core, leading to pad instability, insufficient absorption and leaking.

The elastic members may comprise a single elasticated tape, but more conveniently may comprise a plurality of elasticated threads.

Preferably each of the four elastic members also extends through a respective section of the front or the rear section of the disposable underpants. In this way the disposable underpants are elasticated over and around the hip area to provide an improved fit on the user. Conveniently, each elastic member extends from a turning point situated midway between the sides of either the front section or the rear section to a respective one of the four sides.

In one embodiment of the present invention the disposable underpants are formed in a continuous web in conventional fashion and each of the said four groups of elastic members is provided around a respective quadrant of each opening cut in the web between adjacent underpants to form the cut outs therein along the sides of the crotch sections which form the leg openings. In an alternative embodiment each of the said four groups of elastic members is provided around a respective two quadrants of every alternate opening cut in the web.

An embodiment of the present invention will now be described, by way of example, with reference to the accompanying drawings, in which:
Fig. 1 shows an exploded perspective view of disposable underpants in accordance with the present invention;
Fig. 2 shows a plan view of a continuous web of disposable underpants in accordance with a first embodiment of the present invention; and
Fig. 3 shows a plan view of a continuous web of disposable underpants in accordance with a second embodiment of the present invention.

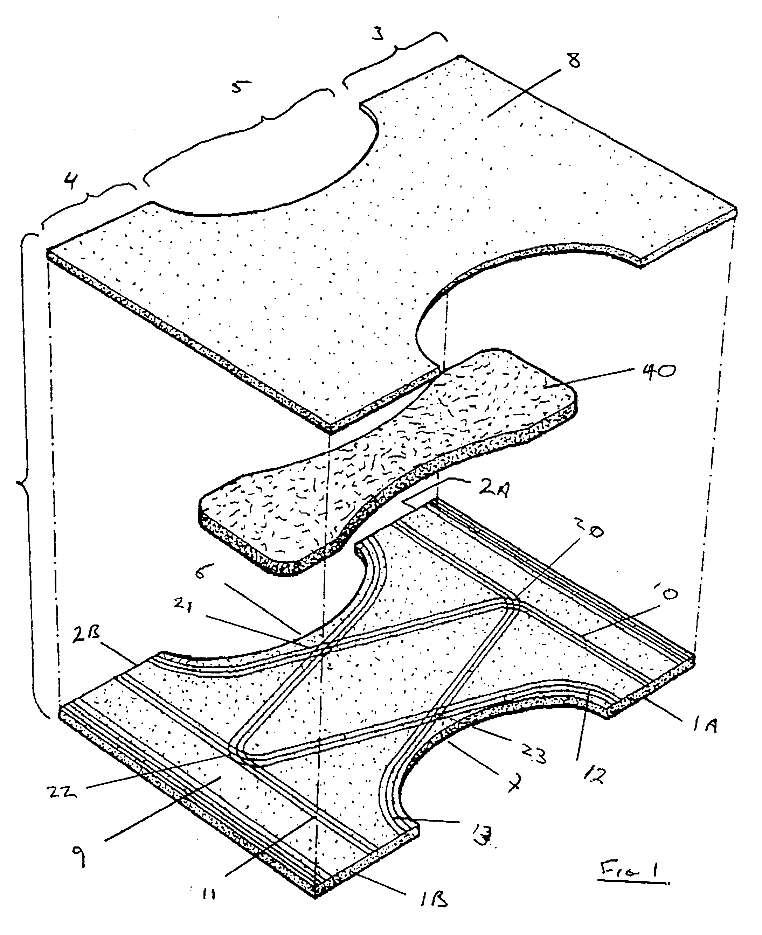

Referring to Fig. 1 of the drawings there is shown an exploded perspective view of disposable underpants in accordance with the present invention. As shown the underpants have not been joined together at the sides 1A, 1B and 2A, 2B, ready for use. For ease of understanding the underpants may be considered to comprise three distinct regions; a front section 3, a rear section 4 and a crotch section 5 connecting the front and rear sections 3, 4 together. A cut out is formed down each side 6 and 7 of the crotch section 5 and these cut outs define the leg openings of the underpants.

The underpants comprise a top sheet 8, a back sheet 9 and a core of water absorbent material 10 sandwiched between the top and back sheets 8, 9. The choice of materials and the method of production of the underpants are essentially conventional and need not be described in detail herein. The principle point of difference over conventional disposable underpants lies in the elastication which is provided around the leg openings and the hip openings. This elastication will now be described in detail with reference to Fig. 1.

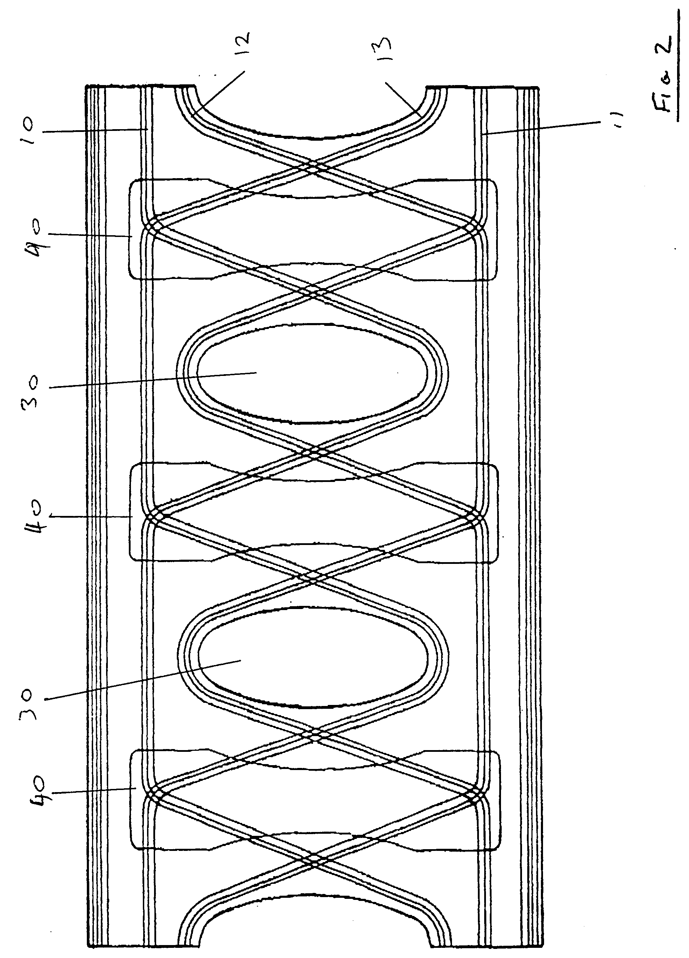

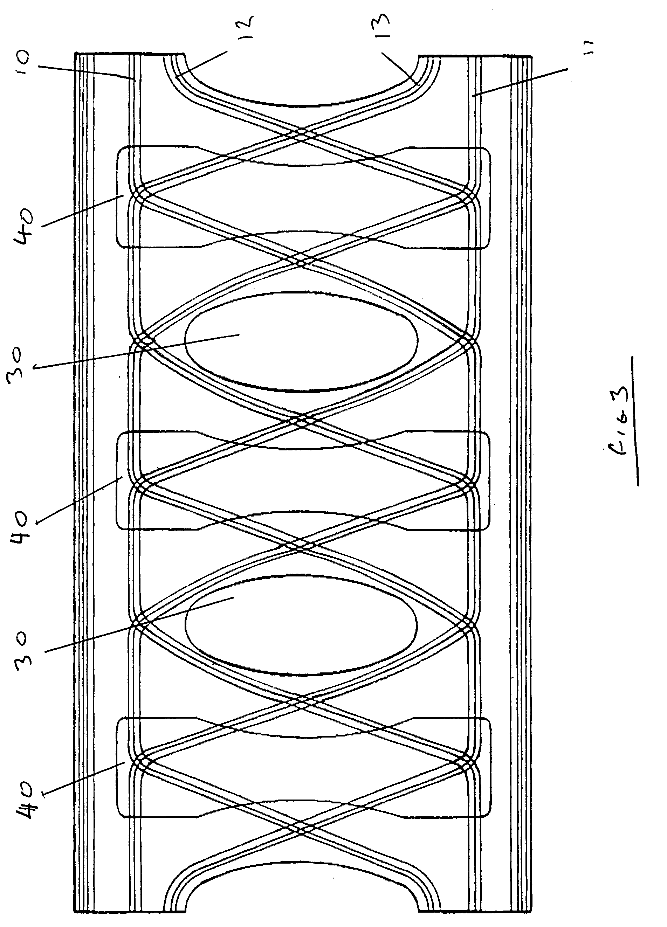

The elastication in the disposable underpants comprises four groups of elastic threads 10, 11, 12 and 13. The threads in each group run parallel to one another through the underpants and are bonded in place to the backsheet. At this point it will be useful to explain that disposable underpants of this type are usually manufactured in a continuous web as shown in Figs. 2 and 3 from a continuous backsheet web, a continuous topsheet web and continuous elastic threads. An absorbent pad 40 is inserted at regular intervals between the topsheet web and the backsheet web, and an opening 30 is cut between every adjacent pair of absorbent pads 40. Each opening 30 forms a pair of the cut outs which define the sides of the crotch sections and there by the leg openings. As production of the leading underpants in the continuous web is completed the continuous web is folded in half longitudinally through the crotch section 5 to bring the sides 1A, 1B and 2A, 2B together. The sides are then ultrasonically sealed together and the finished underpants, ready for wear, are detached from the continuous web. As the continuous backsheet web travels forward each of the four groups of elastic threads 10, 11, 12 and 13 is applied thereto by a respective shuttle mechanism. In this regard this method of manufacture is identical to that suggested in US-A-5092861 except that there are four shuttle mechanisms instead of two.

As can be seen in Figs. 2 and 3 each group of threads 10, 11, 12 and 13 follows an essentially sinusoidal path back and forth across the full width of the continuous web, but is out of phase with each of the other three. In a single pair of the underpants as shown in Fig. 1 these paths are as follows.

The group of threads 10 enter the underpants at a point on the side 1A of the front section 3 and extend in a longitudinal direction to a turning point 20 situated half way across the front section 3. Here they change direction to pass transversely across the crotch section 5 to the rear section 4. As the group of threads 10 passes the midway point 21 in the side 6 of the crotch section 5 they curve to follow the edge thereof and finally exits the underpants at a point in the side 2B of the rear section 4.

The group of threads 11 follow an identical path, but exactly 180° out of phase. This means that the group of threads 11 enter the underpants at a point on the side 1B of the rear section 4 and exit the underpants at a point on the side 2A of the front section 3. In between the threads pass through a turning point 22 situated half way across the rear section 4, intersect the group of threads 10 at the midway point 6 in the side 5 of the crotch section 5 and follow the other half of the edge thereof from the group of threads 10.

The group of threads 12 also follows an identical path to that of the group of threads 10, but in reverse. That is to say the threads enter the underpants at a point in the side 1A of the front section 3 and follow the edge of the side 7 of the crotch section 5 to the middle 23 thereof. The threads then extend to the turning point 22 halfway across the rear section 4 where they intersect with the threads of group 11 and then change direction to follow a longitudinally extending path which exits the underpants at a point in the side 2B of the rear section 4.

Lastly, the group of threads 13 follows an identical path to that of the threads 12, but exactly 180° out of phase. This takes the threads 13 around the other half of the edge of side 7 of the crotch section 5 and out at a point in the side 2A of the front section 3. The group of threads 13 intersect with group 12 at point 23 and with group 10 at point 20.

Figs. 2 and 3 of the drawings illustrate alternative paths which can be followed by each of the four groups of threads through the continuous web. In Fig. 2 each group of threads is provided around two quadrants or one half of every alternate opening cut in the continuous web. In Fig. 3 each group of threads is provided around a respective quadrant of each opening cut in the continuous web.

The pattern of the elastication adopted in the disposable underpants of the present invention ensures that each leg open is completely surrounded to ensure a snug and fluid tight fit. However, because there are no elastic members extending transversely through the crotch area there are no elastic forces acting to pull in the sides of the crotch section which might lead to leakage. Moreover, for the same reason there is no likelihood of bunching of the absorbent core which can lead to pad instability, resulting in insufficient absorption and leaking. Indeed the pattern of elastication effectively encloses the working area of the core between the elastic threads extending between four points of intersection 20, 21, 22 and 23 then providing a double anti leak seal in this important region.

## Claims

1. Disposable pants comprising a front section (3), a rear section (4), a crotch section (5) which connects the front and rear sections (3 and 4) together and the sides (6 and 7) of which are cut away to define leg openings, and elastication which extends along the sides of each cut away so as to elasticate the leg openings and ensure a secure and fluid tight fit on a wearer, characterised in that the elastication comprises four groups of elastic members (10, 11, 12 and 13); and
one of the four groups of elastic members (12) extends from a point on one side (1A) of the front section (3) to a point on the diametrically opposite side (2B) of the rear section (4) along the front half of one of the two cut aways which define the leg openings;
another of the four groups of elastic members (10) extends from a point on the said one side (1A) of the front section (3) to a point on the said diametrically opposite side (2B) of the rear section (4) along the rear half of the other of the two cut aways which define the leg openings;
yet another of the four groups of elastic members (13) extends from a point on the side (1B) of the rear section (4) directly opposite the said one side (1A) of the front section (3) to a point on the diametrically opposite side (2A) of the front section (3) along the rear half of the said one of the two cut aways which define the leg openings; and
the last of the four groups of elastic members (11) extends from a point on the said side (1B) of the rear section (4) directly opposite the said one side (1A) of the front section (3) to a point on the diametrically opposite side of the front section along the front half of the said other of the two cut aways which define the leg openings.

2. Disposable pants according to claim 1, characterised in that the elastic members comprise a single elasticated tape.

3. Disposable pants according to claim 1, characterised in that the elastic members comprise a plurality of elasticated threads.

4. Disposable pants according to claim 1, 2 or 3, characterised in that each of the four elastic members comprises a portion which extends from a respective one of the sides of the front and rear sections to a point midway between that side and the opposite side.

5. A continuous longitudinal web of disposable pants, each of the pants comprising a front section, a rear section and a crotch section which connects together the front and rear sections, and being .PA connected side by side in the web, in which a hole is cut in the web between the crotch sections of each adjacent pair of pants to form respective leg openings and elastication is provided around the holes to provide leg elastication, characterised in that the elastication comprises four groups of elastic members, each of which describes a substantially sinusoidal path through the web such that a portion of each group extends along a respective one of the front and rear halfs of each of the two leg openings in each of the pants.

6. A continous web according to claim 5, characterised in that each of the said four groups of elastic members extends around a respective quadrant of each of the said holes cut in the web, the said quadrant defining a front or a rear half of one of the leg openings defined by each hole.

7. A continous web according to claim 5, characterised in that each of the said four groups of elastic members extends around a respective two quadrants of every alternate hole cut in the web, the said two quadrants forming the two front or the two rear halfs of the two leg openings defined by each hole.
